# EUROPEAN PATENT SPECIFICATION

(11) **EP 0 561 983 B1**
(45) Date of publication and mention of the grant of the patent: **17.06.1998**
(21) Application number: 92902108.7
(22) Date of filing: 06.12.1991
(51) Int. Cl.: A61F 13/02, A61F 13/00

(54) **SYSTEMS FOR ADMINISTERING DRUGS TRANSDERMALLY USING SORBITAN ESTERS AS SKIN PERMEATION ENHANCERS**
VORRICHTUNG ZUM TRANSDERMALEN VERABREICHEN VON MEDIKAMENTEN UNTER VERWENDUNG VON SORBITANESTERN ZUM VERBESSERN DER HAUTDURCHLÄSSIGKEIT
DISPOSITIFS D'ADMINISTRATION TRANSDERMIQUE DE MEDICAMENTS AU MOYEN D'ESTERS DE SORBITANE EN TANT QU'AGENTS D'ACCENTUATION DE LA PERMEABILITE EPIDERMIQUE

(30) Priority: 10.12.1990 US 625906
(43) Date of publication of application: 29.09.1993
(73) Proprietor: THERATECH, INC., Salt Lake City Utah 84108 (US)
(72) Inventor: EBERT, Charles, D., Salt Lake City, UT 84103 (US); PATEL, Dinesh, Murray, UT 84107 (US); HEIBER, Sonia, Salt Lake City, UT 84108 (US)
(74) Representative: Goldin, Douglas Michael
(86) International application number: PCT/US91/09215
(87) International publication number: WO 92/10154

(56) References cited:
- EP-A- 0 152 281
- EP-A- 0 328 806
- EP-A- 0 413 034
- WO-A-90/11065
- US-A- 4 685 911
- US-A- 4 751 087
- JOURNAL OF PHARMACOBIO-DYNAMICS vol. 9, no. 6, June 1986, pages 517 - 525 OGISO T. ET AL 'ABSORPTION OF INDOMETHACIN AND ITS CALCIUM SALT TROUGH RAT SKIN : EFFECT OF PENETRATION ENHANCERS AND RELATIONSHIP BETWEEN IN VIVO AND IN VITRO PENETRATION'
- INTERNATIONAL JOURNAL OF PHARMACEUTICS vol. 33, no. 1-3, November 1986, pages 225 - 234 AUNGST B. ET AL 'ENHANCEMENT OF NALOXONE PENETRATION TROUGH HUMAN SKIN USING FATTY ACIDS , FATTY ALCOHOLS , SURFACTANTS , SULFOXIDES AND AMIDES'
- DRUG DEV. AND IND. PHARM. vol. 17, no. 14, 1991, pages 1905 - 1930 YU J. ET AL 'TRANSDERMAL DUAL-CONTROLLED DELIVERY OF TESTOSTERONE AND ESTRADIOL : (II) ENHANCED SKIN PERMEABILITY AND MEMBRANE-MODERATED DELIVERY'
- J. OF PHARM. SCIENCES vol. 78, no. 4, April 1989, pages 319 - 323 OGISO T. ET AL 'A PHARMACOKINETIC MODEL FOR THE PERCUTANEOUS ABSORPTION OF INDOMETHACIN AND THE PREDICTION OF DRUG DISPOSITION KINETICS'
- PHARMAZIE vol. 36, no. 2, 1981, pages 112 - 113 PASICH J. ET AL 'DYNAMIK DER ARZNEIMITTELABGABE AUS SALBENGRUNDLAGEN'
- J. OF PHARM. SCIENCES vol. 65, no. 12, December 1976, pages 1780 - 1782 SHEN W. -W- ET AL 'EFFECT OF NON-IONIC SURFACTANTS ON PERCUTANEOUS ABSORPTION OF SALICYLIC ACID AND SODIUM SALICYLATE IN THE PRESENCE OF DIMETHYL SULFOXIDR'

## Description

### Technical Field

The present invention relates generally to the transdermal administration of pharmacologically active agents.

### Background

The delivery of drugs through the skin provides many advantages; primarily, such a means of delivery is a comfortable, convenient and noninvasive way of administering drugs. The variable rates of absorption and metabolism encountered in oral treatment are avoided, and other inherent inconveniences--e.g., gastrointestinal irritation and the like--are eliminated as well. Transdermal drug delivery also makes possible a high degree of control over blood concentrations of any particular drug.

Skin is a structurally complex, relatively thick membrane. Molecules moving from the environment into and through intact skin must first penetrate the stratum corneum and any material on its surface. They must then penetrate the viable epidermis, the papillary dermis, and the capillary walls into the blood stream or lymph channels. To be so absorbed, molecules must overcome a different resistance to penetration in each type of tissue. Transport across the skin membrane is thus a complex phenomenon. However, it is the cells of the stratum corneum which present the primary barrier to absorption of topical compositions or transdermally administered drugs. The stratum corneum is a thin layer of dense, highly keratinized cells approximately 10-15 microns thick over most of the body.

In order to increase skin permeability, and in particular to increase the permeability of the stratum corneum (i.e., so as to achieve enhanced penetration, through the skin, of the drug to be administered transdermally), the skin may be pretreated with a penetration enhancing agent (or "permeation enhancer", as sometimes referred to herein) prior to application of a drug. Alternatively, and preferably, a drug and a permeation enhancer are delivered concurrently.

The present invention is directed to a novel composition for enhancing the penetration of pharmacologically active agents through skin, the composition based on a sorbitan ester as will be described herein. The composition may or may not contain an aliphatic alcohol as an additional component. The sorbitan ester compositions of the invention have been found by the inventors herein to be particularly effective in enhancing the penetration of pharmaceutically active agents through skin.

While there are a number of patents and publications available which relate to the transdermal administration of drugs and to skin permeation enhancer compositions, applicants are unaware of any art which suggests that sorbitan esters are useful as permeation enhancers in the absence of additional permeation enhancing compounds or which describes the sorbitan ester/aliphatic alcohol compositions as described and claimed herein.

The systems and methods of the present invention are useful for administering a wide variety of drugs and drug types. For example, one focus of the invention is on the enhanced transdermal administration of steroids.

Nitroglycerin was originally administered orally and, later, bucally. More recently, transdermal systems have been developed which have been found to improve the systemic bioavailability of the drug. The Nitro-Dur® Transdermal Infusion System manufactured and sold by Key Pharmaceuticals, Inc. (Kenilworth, New Jersey), the Nitrodisc® made by Searle Pharmaceuticals, Inc. (Chicago, Illinois), the Nitroglycerin Transdermal System of Bolar Pharmaceutical Co., Inc. (Copiague, New York), the Transderm-Nitro® system distributed by CIBA Pharmaceutical Co. (Summit, New Jersey), and the Minitran® system of Riker Pharmaceuticals (St. Paul, Minnesota) are examples of the currently available transdermal nitroglycerin systems.

Several problems which have been encountered with the transdermal administration of nitroglycerin include the tolerance to the drug which results from prolonged administration. Higher and higher doses are required for patients on long-term nitroglycerin therapy, necessitating the use of larger and larger transdermal patches. This is obviously inconvenient and undesirable. Accordingly, the present invention is directed to a method and system for administering nitroglycerin transdermally at a relatively high transdermal flux, using a more conveniently sized, smaller patch. The invention may also be useful to treat indications other than angina pectoris, e.g., congestive heart failure or production of controlled hypotension during surgery and perioperative to surgery, which require higher doses of nitroglycerin. The invention involves the transdermal administration of nitroglycerin in combination with a low irritation skin permeation enhancer as will be discussed in detail herein.

### Citation of Art

The following references relate to one or more aspects of the present invention.

Skin permeation enhancers, generally: Various compounds for enhancing the permeability of skin are known in the art. U.S. Patent Nos. 4,006,218, 3,551,554 and 3,472,931, for example, respectively describe the use of dimethylsulfoxide (DMSO), dimethyl formamide (DMF) and N,N-dimethylacetamide (DMA) to enhance the absorption of pharmacologically active agents through the stratum corneum. Other compounds which have been used to enhance skin permeability include: decylmethylsulfoxide (C₁₀MSO); diethylene glycol monoethyl ether; polyethylene glycol monolaurate (PEGML; see, e.g., U.S. Patent No. 4,568,343); glycerol monolaurate (U.S. Patent No. 4,746,515); propylene glycol monolaurate; ethanol (e.g., as in U.S. Patent No. 4,379,454); eucalyptol (U.S. Patent No. 4,440,777); lecithin (U.S. Patent No. 4,783,450); the l-substituted azacycloheptan-2-ones, particularly l-n-dodecylcyclazacycloheptan-2-one (available under the trademark Azone® from Nelson Research & Development Co., Irvine, CA; see U.S. Patent Nos. 3,989,816, 4,316,893, 4,405,616 and 4,557,934); propylene glycol in combination with a fatty acid such as linoleic acid (European Patent Publication No. 261429); "cell envelope disordering compounds" such as methyl laurate or oleic acid in combination with N-(hydroxyethyl) pyrrolidone (U.S. Patent No. 4,537,776); C₃-C₄ diols (U.S. Patent No. 4,552,872, European Patent Application Publication No. 043738); or a binary system of oleic acid, oleins or oleyl alcohol in combination with a lower alcohol (U.S. Patent No. 4,863,970).

Sorbitan analogs as permeation enhancers, specifically: T. Ogiso et al., J. Pharmacobio-Dyn., 9:517-525 (1986), presents studies on percutaneous absorption in vivo and the penetration in vitro of indomethacin. Sorbitan monooleate was tested as a permeation enhancer in combination with a dimethyl sulfoxide (DMSO) gel and was found to have no enhancing effect. T. Ogiso et al., J. Pharm. Sci., 78(4):319-323 (1989), describes the combined use of laurocapram and sorbitan monooleate in a permeation enhancer composition also containing a DMSO gel, for the transdermal administration of indomethacin. W.-W. Shen et al., J. Pharm. Sci., 65(12):1780-1783 (1986), describes the effect of various nonionic surfactants, including sorbitan monopalmitate and sorbitan trioleate, on the percutaneous absorption of salicylic acid. As with the latter two references, the sorbitan esters are used in conjunction with DMSO. U.S. Patent No. 4,637,930 to Konno et al. describes a transdermal formulation for the administration of nicardipine hydrochloride which contains a mixed liquid composed of urea and an additional compound which may be a sorbitan "middle chain" (6-12 carbon atom) fatty acid ester.

Transdermal administration of nitroglycerin, generally: U.S. Patent No. 4,751,087 to Wick describes an acrylate-based adhesive tape for delivering nitroglycerin transdermally. The system optionally includes as a skin penetration enhancing combination (i) a fatty acid ester, and (ii) glyceryl monolaurate. Patent No. 4,615,699 to Gale et al. describes a transdermal delivery system for administering nitroglycerin in conjunction with ethanol as a skin permeation enhancer. U.S. Patent No. 4,764,381 to Bodor et al. describes nitroglycerin compositions for transdermal administration, containing oleic acid as a permeation enhancer. U.S. Patent No. 4,559,222 to Enscore describes a matrix composition for transdermal drug administration, the matrix containing mineral oil, polyisobutylene, and colloidal silicon dioxide. U.S. Patent No. 4,698,062 to Gale et al. relates to a transdermal drug delivery device which effects "pulsatile" delivery of nitroglycerin to a patient. U.S. Patent No. 4,814,168 to Sablotsky et al. describes transdermal drug delivery systems stated to be useful in the administration of nitroglycerin, the systems containing a "multipolymer" drug reservoir of vinyl acetate, polyethylene, rubber, and a tackifying agent. PCT Publication No. WO83/00093, inventors Keith et al., relates to a polymeric diffusion matrix for the transdermal administration of nitroglycerin which contains two polyvinyl alcohol components and glycerol. PCT Publication No. WO86/00814, inventors Sablotsky et al., relates to an adhesive bilayer transdermal delivery system for the administration of nitroglycerin.

Aungst *et al*, *International Journal of Pharmaceutics* **33**: 225-234 (1986) describes examination of the permeation of the drug naloxone through human skin using a variety of adjuvants in 10% propylene glycol.

Yu *et al, Drug Development and Industrial Pharmacy* **17 (14):**1905-1930 (1991) describes the transdermal delivery of testosterone and estradiol.

EP-A-0413034 refers to plasters containing a knitted fabric and an adhesive layer containing a drug for percutaneous administration.

### Summary of the Invention

It is an object of the invention to provide a transdermal system in the form of a laminated composite designed to adhere to the skin. The composite contains, in addition to the selected pharmacologically active agent to be administered, a permeation enhancer composition containing a sorbitan ester, and, optionally, an aliphatic alcohol.

It is an object of the invention to provide a laminated composite as a transdermal patch which contains drug and the selected permeation enhancer in one or more drug reservoir layers.

It is yet another object of the invention to provide compositions for the enhanced transdermal administration of steroid drugs.

Additional objects, advantages and novel features of the invention will be set forth in part in the description which follows, and in part will become apparent to those skilled in the art upon examination of the following, or may be learned by practice of the invention.

The invention enables the performance of a method for administering a pharmacologically active agent transdermally so as to achieve relatively high transdermal fluxes, by administering, through a predetermined area of intact skin and for a predetermined period of time, (1) the agent, and (2) a permeation enhancer consisting essentially of a sorbitan ester, or a sorbitan ester in combination with a C₁-C₄ aliphatic alcohol. The skin permeation enhancer and the drug are administered in a single composition. As the clearance rate of many drugs from the body is quite high, it is generally preferred that administration be substantially continous throughout the time period chosen for patch application.

In one aspect of the invention, a composition of matter is provided that is useful for the delivery of a pharmacologically active agent through the skin. The invention provides a laminated composite capable of administering a pharmacologically active agent which is a steroid, pindolol or nitroglycerin through a selected area of skin over a sustained time period, the composite comprising:
(a) a backing layer that is substantially impermeable to the pharmacologically active agent;
(b) a matrix layer having a basal surface adapted to be adhered to the skin comprising:
   (i) a pressure-sensitive adhesive;
   (ii) a therapeutically effective amount of the pharmacologically active agent; and
   (iii) a permeation enhancer which is a sorbitan ester the structural formula: 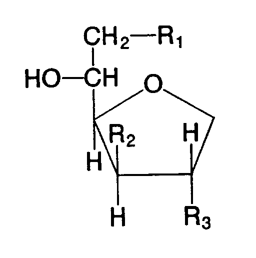 wherein R₁ has the formula -O(CO)R' where R' represents a saturated, mono-unsaturated, di-unsaturated, or tri-unsaturated aliphatic hydrocarbon group of from 7 to 21 carbon atoms optionally containing from 1 to 3 hydroxyl groups;
      and both R₂ and R₃ are hydroxyl groups.

The invention may thus provide a therapeutic system for administering a drug transdermally, at relatively high fluxes as noted above, in the form of a skin patch. The skin patch is in the form of a matrix-type laminated composite containing an upper backing layer that is substantially impermeable to the drug, and at least one drug/enhancer reservoir, one of which forms the basal surface of the device and is designed to adhere to the skin during use. The reservoir is a matrix which contains both the drug and a permeation enhancer as described above. Such a laminated composite preferably includes a strippable protective release liner laminated to the basal surface of the drug reservoir. The release liner is a disposable element designed to protect the exposed reservoir surface prior to use. In an alternative embodiment, a transdermal therapeutic system is provided in the form of a liquid reservoir-type laminated composite, e.g., as described in commonly assigned U.S. Patent No. 4,849,224 to Chang et al..

The invention may thus be used in a method for administering nitroglycerin transdermally so as to achieve relatively high transdermal fluxes, by coadministering, through a predetermined area of intact skin and for a predetermined period of time, (1) nitroglycerin, and (2) a permeation enhancer as described herein. In a preferred embodiment, the skin permeation enhancer and the drug are administered in a single composition which contains both components. As the clearance rate of nitroglycerin from the body is quite high, it is preferred that administration be continuous throughout the time period chosen for patch application.

It should also be noted that the present invention can allow the administration of nitroglycerin to individuals in need of nitroglycerin therapy, generally, and thus encompasses transdermal methods and systems designed for a variety of indications, e.g., the relief and treatment of angina pectoris, congestive heart failure, and the like

The invention may thus provide a therapeutic system for administering nitroglycerin transdermally, at relatively high fluxes as noted above, in the form of a skin patch. The skin patch is a laminated composite containing an upper backing layer that is substantially impermeable to the drug, and at least one drug/enhancer reservoir, one of which forms the basal surface of the device and is designed to adhere to the skin during use. The reservoir contains both the nitroglycerin and a permeation enhancer as described herein.

The laminated composite may further include a strippable protective release liner laminated to the basal surface of the drug reservoir. The release liner is a disposable element designed to protect the exposed reservoir surface prior to use. The release liner, for ease of removal, is preferably a two-part structure in which a first strippable protective sheet partially overlaps a second strippable protective sheet, giving rise to a tab extending from the basal surface of the patch.

### Brief Description of the Drawing

Figure 1 is a schematic sectional view through a laminated matrix-type transdermal system of the invention.

Figure 2 is a schematic sectional view through a laminated liquid reservoir-type transdermal system of the invention.

### Detailed Description of the Invention

Before describing the present composition of the invention in detail, it is to be understood that this invention is not limited to the aliphatic alcohols, or laminate materials described herein as such may, of course, vary. It is also to be understood that the terminology used herein is for the purpose of describing particular embodiments only, and is not intended to be limiting.

It must be noted that, as used in this specification and the appended claims, the singular forms "a," "an" and "the" include plural referents unless the content clearly dictates otherwise. Thus, for example, reference to a laminated structure containing "a drug" includes a mixture of two or more drugs, reference to "an adhesive" includes reference to one or more of such adhesives, and reference to "a sorbitan ester" includes reference to a mixture of two or more sorbitan esters.

In describing and claiming the present invention, the following terminology will be used in accordance with the definitions set out below.

"Penetration enhancement" or "permeation enhancement" as used herein relates to an increase in the permeability of skin to a pharmacologically active agent, i.e., so as to increase the rate at which the agent permeates into and through the skin. A "permeation enhancer" is a material which achieves such permeation enhancement, and a "penetration enhancing amount" of an enhancer as used herein means an amount effective to enhance skin penetration of a selected agent to a desired degree.

The term "drug" or "pharmacologically active agent" as used herein is intended to mean a compound or composition of matter which, when administered to an organism (human or animal) induces a desired pharmacologic and/or physiologic effect by local and/or systemic action. In general, the terms include the therapeutic or prophylactic agents in all major therapeutic/prophylactic areas of medicine. The drugs useful in conjunction with the present invention include beta-blockers such as pindolol, steroids and nitroglycerin.

"Carriers" or "vehicles" as used herein refer to carrier materials without pharmacological activity which are suitable for administration in conjunction with the presently disclosed and claimed compositions, and include any such carrier or vehicle materials known in the art, e.g., any liquid, gel, solvent, liquid diluent, solubilizer, or the like. The carriers and vehicles suitable herein are "pharmaceutically acceptable" in that they are nontoxic, do not interfere with drug delivery, and are not for any other reasons biologically or otherwise undesirable. Examples of specific suitable carriers and vehicles for use herein include water, mineral oil, silicone, inorganic gels, aqueous emulsions, liquid sugars, waxes, petroleum jelly, and a variety of other oils and polymeric materials.

By a "therapeutically effective" amount of a drug or pharmacologically active agent is meant a nontoxic but sufficient amount of the drug or agent to provide the desired therapeutic effect.

"Nitroglycerin" shall mean 1,2,3-propanetriol trinitrate, i.e., the compound having the structural formula

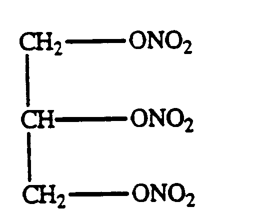

The invention may thus find use in a method for enhancing the rate of penetration of a pharmacologically active agent through the skin, wherein the method involves co-administration of the agent through a predetermined area of intact skin, and for a predetermined period of time, the selected agent and a permeation enhancer consisting essentially of a sorbitan ester or a sorbitan ester in combination with a C₁-C₄ aliphatic alcohol. The sorbitan esters which are useful in conjunction with the present invention have the structure 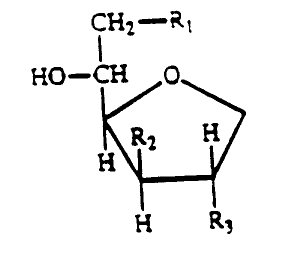 wherein the substituent R₁ has the structure -O(CO)R', where R' is selected from the group consisting of saturated, mono-unsaturated, di-unsaturated and tri-unsaturated aliphatic hydrocarbon substituents of 7 to 21 carbon atoms, preferably 11 to 21 carbon atoms, and may be substituted with 1 to 3 hydroxyl groups. The substituents R₂ and R₃ are hydroxyl. R₁, R₂ and R₃, may be, for example, lauryl, myristyl, palmityl, stearyl, palmitoleyl, oleyl, linoleyl, linolenyl, or ricinoleyl esters, or the like. Exemplary sorbitan esters are long-chain sorbitan monoesters, wherein R₁ is as defined above, R' is a hydrocarbon of 11 to 21 carbon atoms, and R₂ and R₃ are both hydroxyl. Particularly preferred compounds within the class of sorbitan monoesters are sorbitan monooleate and sorbitan monolaurate.

In addition to a sorbitan ester, the permeation enhancer composition of the invention may also include an aliphatic alcohol component. Preferred aliphatic alcohols are the C₁-C₄ alkanols such as ethanol, n-propanol, isopropanol, t-butanol, and mixtures thereof. However, longer chain alcohols (typically C₈-C₂₂) such as oleyl alcohol may also be used (see, e.g., Examples 8 and 9) herein.

The amount of enhancer will typically be in the range of 0.1 wt.% to 40 wt.% relative to the total composition, more preferably on the order of about 2.5 wt.% to 15 wt.%. The composition may, in addition to drug and enhancer, include one or more selected carriers or excipients, and/or various agents and ingredients commonly employed in dermatological ointments and lotions. For example, fragrances, opacifiers, preservatives, antioxidants, gelling agents, perfumes, thickening agents, stabilizers, surfactants, emollients, coloring agents, and the like may be present so long as they are pharmaceutically acceptable and compatible with the drug and enhancer.

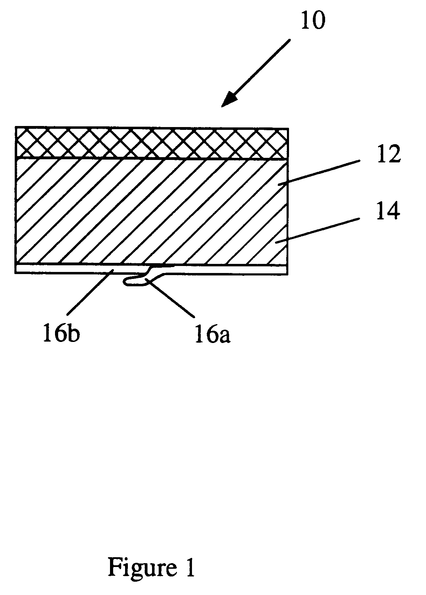

A transdermal delivery system for the administration of a drug can be constructed with the drug/enhancer composition described hereinabove. Transdermal drug delivery systems for use herein are laminated composites which contain one or more drug/permeation enhancer reservoirs, a backing layer and, optionally, one or more additional layers (e.g., additional drug and/or enhancer reservoirs) as those skilled in the art of transdermal drug delivery will readily appreciate. Figure 1 depicts an exemplary system, generally designated **10**, that when applied to skin administers a selected pharmacologically active agent as outlined above. System **10** is a laminated composite in which the top layer **12** is a backing layer, its face forming the top surface of the composite. The drug reservoir, containing drug, enhancer as described herein, and optional carriers or vehicles, is shown at **14**, immediately below and adjacent to the backing layer. Prior to use, the laminate also includes a strippable protective release liner. In a preferred embodiment, as described in commonly assigned US-A-5202125 filed 10 December 1990, and entitled "Method and Systems for Administering Nitroglycerin Transdermally at Enhanced Transdermal Fluxes," the release liner is in the form of two sheets **16a** and **16b**, the first sheet **16a** partially overlapping the second sheet **16b**. Additional structural layers and/or additional drug/enhancer reservoirs may also be present.

The drug reservoir is comprised of a contact adhesive which is a pressure-sensitive adhesive suitable for long-term skin contact. It must also be physically and chemically compatible with the drug and enhancer employed, and with any carriers or vehicles incorporated into the drug/enhancer composition. Further, the adhesive selected for use as the reservoir layer must be such that the drug and enhancer are at least somewhat soluble in the adhesive. The drug reservoir will generally be in the range of about 2 to 4 mils in thickness. Suitable adhesives for use as the drug reservoir include polysiloxanes, polyacrylates, polyurethanes, tacky rubbers such as polyisobutylene, and the like. Particularly preferred contact adhesives for use as the drug reservoir herein are cross-linked acrylates (e.g., the Durotak® adhesives, available from National Starch & Chemical Co., New York, NY, or the Gelva® adhesives, available from Monsanto Co., St. Louis, MO).

The backing layer, which is, as shown, adhered to the drug reservoir and serves as the upper layer of the device during use, functions as the primary structural element of the device. The backing layer is made of a sheet or film of a preferably flexible elastomeric material that is substantially impermeable to the drug/enhancer composition. The layer will typically be on the order of 1.0 to about 4.0 mils in thickness, and is preferably of a material that permits the device to follow the contours of the skin, such that it may be worn comfortably on any skin area, e.g., at joints or other points of flexure. In this way, in response to normal mechanical strain, there is little or no likelihood of the device disengaging from the skin due to differences in the flexibility or resiliency of the skin and the device. Examples of polymers useful for the backing layer herein are polyethylene, polypropylene, polyesters, polyurethanes, polyethylene vinyl acetate, polyvinylidene chloride, block copolymers such as PEBAX, and the like. The backing layer may also comprise laminates of one or more of the foregoing polymers.

The release liner is a disposable element which serves only to protect the device prior to application. Typically, the release liner is formed from a material impermeable to the drug, vehicle, and adhesive, and which is easily stripped from the contact adhesive that serves as the drug reservoir layer. Preferred release liners for use herein are those which are generally suitable for use in conjunction with pressure-sensitive adhesives. Silanized polyester films are presently preferred.

In a preferred embodiment, as noted above, a two-part release liner is used, wherein a first strippable protective sheet (shown as **16a** in Figure 1) partially overlaps a second strippable protective sheet **16b**, such that the area of overlap gives rise to a tab which extends from the basal surface of the laminate, enabling ready removal of the strippable sheets from the reservoir layer.

The laminated composites of the invention are as shown in Figure 1, having a backing layer, a drug reservoir, and a two-piece release liner; the drug reservoir contains a drug/enhancer composition as described above, with the quantity of the drug therein, and with the remainder of the drug reservoir comprised of adhesive and optional carriers, vehicles or the like. To use these laminated composites, one is applied directly to the skin of a patient, to release the drug/enhancer composition to the skin, allowing the drug to permeate into the circulation. The adhesive layer which serves as the drug reservoir should be in firm contact with the skin.

In general, such devices are fabricated using methods standard in the art, e.g., solvent-evaporation film casting in which all components of the drug/enhancer composition are admixed with the adhesive which will serve as the drug reservoir, and cast onto a substrate which is either the backing layer or release liner. Other layers are then laminated to this initial structure.

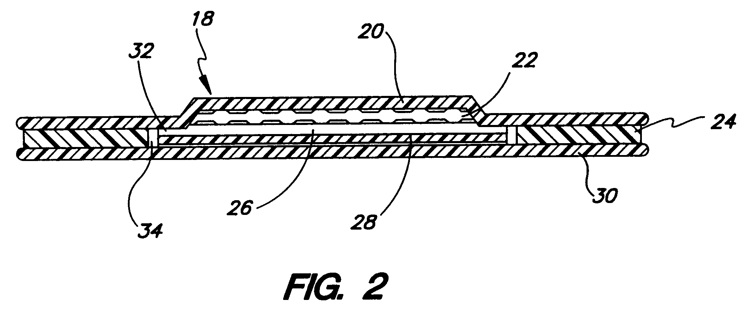

In an alternative embodiment, laminated composites containing drug in a liquid reservoir, as described in U.S. Patent No. 4,849,224, may be used. As described in that patent, such devices shown generally at **18** in Figure 2 are comprised of an uppermost layer of a heat-sealable backing film **20** having an inverted, cup-shaped recess that serves the reservoir **22** for the drug-enhancer formulation. The underside of the outer edge of the backing film carries a ring-shaped layer **24** of a pressure-sensitive adhesive peripheral to the reservoir. Underlying the reservoir, just inward of the peripheral ring of adhesive, is a membrane layer **26** that is permeable to the drug-enhancer formulation. A peel sealable inner liner **28** underlies membrane **26** and portions of backing film **20**. A peel-sealable release liner **30** covers the entire underside of the assembly and forms the basal surface of the device. Device **18** has a heat seal **32** between the membrane and backing film and a peelable (impermanent) heat seal **34** between the backing film and the inner liner **28**. An alternative liquid reservoir-type device which may be used in conjunction with the present compositions is described in U.S. Patent No. 4,983,395.

Preferred daily dosages obtained with the present methods and systems will, similarly, vary with the drug administered. The targeted daily dosage will depend on the individual being treated, the indication addressed, the length of time the individual has been on the drug, and the like.

Turning now to the administration of nitroglycerin, specifically, it is preferred that the drug and the selected permeation enhancer or enhancers are administered to the individual simultaneously, in a single composition. As with other drugs, a preferred mode of administration, however, is via a transdermal patch, wherein the preferred quantities of the various components will be outlined below.

In a preferred embodiment, the nitroglycerin and the selected permeation enhancer or enhancers are administered such that flux is increased by at least about 50% relative to that which would be achieved in the absence of a permeation enhancer, that is, typically, a flux on the order of at least about 10 µg/cm²/hr, preferably at least about 20 µg/cm²/hr, most preferably at least about 30 µg/cm²/hr, is achieved. It will be appreciated by those skilled in the art of transdermal drug delivery that the foregoing numbers are approximate, as transdermal flux may vary with the individual undergoing treatment as well as the particular skin site chosen for transdermal drug delivery.

It is also preferred that the time period for nitroglycerin administration be on the order of 12 to 36 hours, optimally about 12 to 24 hours, during which time drug delivery is substantially continuous.

Preferred daily dosages obtained with the present methods and systems are typically in the range of 2.5 to 50 mg, more typically in the range of about 2.5 to 20 mg, for nitroglycerin. The targeted daily dosage will, however, depend on the particular drug being administered, the individual being treated, the indication addressed, the length of time the individual has been on the drug, and the like. Generally, and as noted above, the present methods and systems enable transdermal administration of nitroglycerin at somewhat higher transdermal fluxes than enabled with the majority of previously known transdermal nitroglycerin systems, and may thus be useful for treating disorders which require a higher daily dosage than is typical (e.g., congestive heart failure), or for treating patients who have developed some tolerance for the drug. The invention also enables the use of conveniently sized small patches.

Transdermal delivery of nitroglycerin with the permeation enhancers of the invention enable the use of a skin area for drug administration in the range of approximately 1.0 cm² to about 100 cm². At higher transdermal fluxes, smaller skin areas within the aforementioned range may be targeted, i.e., less than about 25 cm² or, more preferably, less than about 10 cm².

### Experimental

The following examples are put forth so as to provide those with ordinary skill in the art with a complete disclosure and description of how to formulate compositions and systems of the invention, and are not intended to limit the scope of what the inventors regard as their invention. Efforts have been made to ensure accuracy with respect to numbers used (e.g., amounts, temperatures, etc.), but some experimental errors and deviations should be allowed for. Unless indicated otherwise, parts are parts by weight, temperatures are in degrees Centigrade, and pressure is at or near atmospheric.

Estradiol, norethindrone acetate, progesterone and pindolol were obtained from Sigma Chemical Co., St. Louis, MO. Polyacrylate adhesive solutions were obtained from National Starch & Chemical Co., New Jersey (Durotak® 80-1194, 80-1054, 80-1070) and from Monsanto Corporation, St. Louis, MO (Gelva® 737). Sorbitan monooleate, sorbitan monolaurate and sorbitan trioleate were all obtained from ICI Americas. Ethanol (USP 95%) was obtained from Fisher Scientific.

Adhesive laminates were formulated by mixing the selected polyacrylate solutions with drug and/or enhancer, followed by evaporation of solvent. The concentrated solution was cast onto the silanized surface of a polyester release liner (Release Technologies, 2-EST-A-S242M), using a 10 mil gap Gardner knife. The cast adhesive was then dried at 80°C for 15 minutes in a convection oven to yield a final 0.050 inch thick adhesive coating. A 0.0075 inch thick low density polyethylene film (Schoeller Technical Paper Co., New York) was then laminated onto the dried adhesive surface to produce a three-layer transdermal matrix system construction.

The in vitro skin flux of the particular drugs tested was evaluated across human cadaver skin as described by Merrit and Cooper (J. Controlled Release (1984) 1:161) using a high-performance liquid chromatography (HPLC) assay. For these studies the release liner was removed from a previously cut section of the above transdermal matrix construction. The adhesive matrix was then positioned onto the stratum corneum surface of heat separated human epidermis and the skin, with the adhering transdermal system, was then immediately mounted onto the diffusion cell. The steady state flux (µg/cm²/hr) of drug was determined by linear regression analysis of the cumulative amount of drug permeating (µg/cm²) across the skin as a function of the time (hr).

### Example 1

The aforementioned procedure was used to evaluate the effect of increasing sorbitan ester levels on estradiol flux from acrylic adhesives. The sorbitan ester used was sorbitan monolaurate ("SML"); the acrylic adhesive used was Durotak® 80-1194 ("1194").

Results are set forth in Table I. As may be seen, the flux obtained was found to increase with increasing quantities of sorbitan monolaurate.

**Table I**

| Effect of Increasing Sorbitan Monolaurate Levels on Estradiol Flux from an Acrylic Adhesive | | |
|---|---|---|
| Enhancer System | Estradiol Conc. (mg/ml) | Flux (µg/cm²/hr) |
| None | 2 wt.% | 0.26 ± 0.05 |
| 5 wt.% SML | 2 wt.% | 0.34 ± 0.09 |
| 10 wt.% SML | 2 wt.% | 0.43 ± 0.08 |

### Example 2

The procedure of Example 1 was followed to evaluate the effect of sorbitan monooleate ("SMO") and sorbitan monolaurate on estradiol flux from three different acrylic adhesive matrices, Durotak® 80-1194 ("1194"), Durotak® 80-1054 ("1054") and Durotak® 80-1070 ("1070"). All systems tested contained 4 wt.% estradiol. As may be deduced from the results set forth in the following table, the two sorbitan esters were found to significantly increase flux in all three types of adhesive matrices.

**Table II**

| Effect of Sorbitan Esters on Estradiol Flux From Three Different Acrylic Adhesive Matrices | |
|---|---|
| Formulation | Flux (µg/cm²/hr) |
| 1194 | 0.38 ± 0.36 |
| 1194/15% SMO | 1.69 ± 0.58 |
| 1194/15% SML | 0.96 ± 0.47 |
| 1054 | 0.55 ± 0.12 |
| 1054/15% SMO | 0.95 ± 0.40 |
| 1054/15% SML | 1.07 ± 0.07 |
| 1070 | 0.41 ± 0.07 |
| 1070/15% SMO | 0.68 ± 0.13 |
| 1070/15% SMO | 0.99 ± 0.31 |

### Example 3

The procedure above was followed to prepare additional acrylic adhesive matrices containing estradiol (both with and without a sorbitan ester), so as to evaluate the effect of drug loading on estradiol flux. The acrylic adhesive used was Durotak® 80-1070 ("1070"), and the sorbitan ester used was sorbitan monooleate. Results are set forth in Table III. Sorbitan monooleate was found to increase estradiol flux in both of the systems tested.

**Table III**

| Effect of Drug Loading on Estradiol Flux from an Acrylic Adhesive Matrix With and Without Sorbitan Monooleate | | |
|---|---|---|
| Formulation | Skin 1 (µg/cm²/hr) | Skin 2 (µg/cm²/hr) |
| 1 wt.% estradiol, no enhancer | 0.22 ± 0.08 | 0.34 ± 0.03 |
| 2 wt.% estradiol, no enhancer | 0.56 ± 0.08 | 0.72 ± 0.09 |
| 1 wt.% estradiol, 5 wt.% SMO | 0.43 ± 0.18 | 0.55 ± 0.07 |
| 2 wt.% estradiol, 5 wt.% SMO | 1.00 ± 0.16 | 0.98 ± 0.08 |

### Example 4

The procedure above was followed to prepare additional acrylic adhesive matrices (Durotak® 80-1194) containing estradiol. One system was prepared with sorbitan trioleate ("STO") as an enhancer, and a second system was prepared without sorbitan trioleate. The flux obtained with the sorbitan trioleate system was approximately 52% higher than that obtained with the control system. Results are set forth in Table IV.

**Table IV**

| Effect of Sorbitan Trioleate on Estradiol Flux (µg/cm²/hr) from an Acrylic Adhesive Matrix | |
|---|---|
| Formulation | Flux (µg/cm²/hr) |
| 4 wt.% estradiol, no enhancer | 0.78 ± 0.13 |
| 4 wt.% estradiol, 10 wt.% STO | 1.19 ± 0.13 |

### Example 5

The procedure above was followed to prepare acrylic adhesive matrices (Durotak® 80-1194 and 80-1054) containing 10 wt.% norethindrone acetate. Systems were prepared with and without 15 wt.% sorbitan ester; both sorbitan monooleate and sorbitan monolaurate were tested, as shown in Table V. As may be concluded from the results summarized in the table, both sorbitan monooleate and sorbitan monolaurate significantly increased norethindrone acetate flux in both adhesive systems.

**Table V**

| Effects of Sorbitan Esters on Norethindrone Acetate Flux from Acrylic Adhesives Matrices | | |
|---|---|---|
| Formulation | Skin 1 (µg/cm²/hr) | Skin 2 (µg/cm²/hr) |
| 1194/no enhancer | 0.21 ± 0.02 | 0.24 ± 0.02 |
| 1194/15% SMO | 0.38 ± 0.20 | 0.31 ± 0.01 |
| 1194/15% SML | 0.43 ± 0.09 | 0.60 ± 0.03 |
| 1054/no enhancer | - | 0.27 ± 0.06 |
| 1054/15% SMO | - | 0.40 ± 0.11 |
| 1054/15% SML | - | 0.54 ± 0.06 |

### Example 6

The procedure above was followed to prepare acrylic adhesive matrices using Gelva® 737 ("737") containing 5 wt.% pindolol. Systems were prepared with 15 wt.% sorbitan monooleate, 15 wt.% sorbitan monolaurate, and 15 wt.% sorbitan trioleate, and compared with a system not containing any sorbitan ester. Again, the flux of drug was found to be significantly higher for all of the systems formulated with the sorbitan esters, relative to the control. Results are set forth in Table VI.

**Table VI**

| Effects of Sorbitan Esters on Pindolol Flux from Acrylic Adhesive Matrices | |
|---|---|
| Formulation | Flux (µg/cm²/hr) |
| 737/no enhancer | 0.23 ± 0.05 |
| 737/15% SMO | 0.55 |
| 737/15% SML | 0.75 ± 0.26 |
| 737/15% STO | 0.55 ± 0.19 |

### Example 7

Flux of progesterone from ethanolic solutions of the drug was evaluated as follows.

Ethanol/water/glycerin/sorbitan ester ointments were prepared as summarized in Table VII, and the flux of the drug therefrom evaluated.

**Table VII**

| Progesterone Flux From Ethanolic Solutions Containing Sorbitan Ester | | | | | |
|---|---|---|---|---|---|
| EtOH | H2O | Glycerin | SMO | SML | Flux µg/cm²/hr |
| -- | 88 | 10 | 2 | -- | 0.61 ± 0.03 |
| -- | 88 | 10 | -- | 2 | 1.21 ± 0.19 |
| 60 | 30 | 10 | -- | -- | 6.29 ± 0.77 |
| 60 | 28 | 10 | 2 | -- | 13.04 ± 1.52 |
| 60 | 28 | 10 | -- | 2 | 10.33 ± 4.02 |

The data summarized in Table 7 clearly demonstrates a synergistic enhancement for progesterone when ethanol is combined with sorbitan monolaurate or sorbitan monooleate.

### (Comparative) Example 8

Nitroglycerin in ethanol (10 vol.%) was obtained from ICI Americas, Inc., Delaware; polyacrylate adhesive solution (Durotak® 80-1194) was obtained from National Starch & Chemical Co., New Jersey. Oleyl alcohol was obtained from Henkel Corp., La Grange, Illinois (HD Eutanol, ultra pure grade). The polyacrylate adhesive solution, nitroglycerin solution and oleyl alcohol were mixed in the required proportion to provide a final solution which upon solvent evaporation would contain 37.5 wt.% nitroglycerin, 5 wt.% oleyl alcohol and 57.5 wt.% polyacrylate adhesive. This solution was rotoevaporated to increase its viscosity to facilitate casting as a film. The concentrated solution was cast onto the silanized surface of a polyester release liner (Release Technologies, 2-EST-A-S242M), using a 10 mil gap Gardner knife. The cast adhesive was then dried at 80°C for 15 minutes in a convection oven to yield a final 0.0025 inch thick adhesive coating. A 0.0025 inch thick low density polyethylene film (Schoeller Technical Paper Co., New York) was then laminated onto the dried adhesive surface to produce a three-layer transdermal matrix system construction. Following the same procedure outlined above, another transdermal matrix system with the final composition of 35 wt.% nitroglycerin and 65 wt.% polyacrylate adhesive was prepared. This matrix did not contain any enhancer.

As controls, the flux of nitroglycerin from an unenhanced nitroglycerin patch (NitroDur II, Key Pharmaceuticals) and an enhanced nitroglycerin patch (Minitran®, Riker Pharmaceuticals) were evaluated in parallel.

**Table VIII**

| HPLC Assay Method for Nitroglycerin | |
|---|---|
| Mobile Phase: | 50% (vol.) acetonitrile |
| | 50% (vol.) water |
| Flow Rate: | 1.3 ml/minute |
| Column: | Partisphere C₁₈ (Whatman) |
| Injection Volume: | 20 µl |
| Wave Length: | 210 nm |
| Retention Time: | 3.0 minutes |
| Run Time: | 5.0 minutes |

Table IX provides the skin flux in two different experiments obtained from the controls and the transdermal matrix patches prepared according to the method described above. The flux obtained from the oleyl alcohol matrix is much higher than that from the unenhanced NitroDur II control patch and the unenhanced nitroglycerin patch prepared as described above. Furthermore, the oleyl alcohol matrix produced significantly higher nitroglycerin skin flux than the enhanced control patch, Minitran.

**Table IX**

| Nitroglycerin Skin Flux (µg/cm²/hr)^{a} | | |
|---|---|---|
| Formulation | Skin 1 | Skin 2 |
| NitroDur II | 20.58 ± 2.60 | 29.92 ± 4.02 |
| Minitran | 26.63 ± 5.92 | 36.69 ± 2.91 |
| 65/35^{b} | 24.21 ± 2.84 | 30.68 ± 2.80 |
| 57.5/37.5/5^{c} | 38.56 ± 0.96 | 45.63 ± 4.33 |

| | | |
|---|---|---|
| ^{a} N = 4, mean ± standard deviation | | |
| ^{b} weight percent of adhesive/nitroglycerin (no enhancer) | | |
| ^{c} weight percent of adhesive/nitroglycerin/oleyl alcohol | | |

### Example 9

Nitroglycerin was premixed into the National Starch Durotak® 80-1194 polyacrylate adhesive by Atlas Powder Company to provide a final solution containing 22.1 wt.% nitroglycerin, 35.8 wt.% solid acrylic adhesive and, 42.1 wt.% total solvents. Oleyl alcohol (Henkel) or sorbitan monooleate ("Arlacel® 80"; ICI Americas) was then dissolved into the nitroglycerin/adhesive solution at various levels to provide final matrix compositions ranging from 0 to 7.5 wt.% enhancer upon drying. These solutions were then cast onto silanized release liners, dried at 80°C for 15 minutes, and finally laminated onto the polyethylene backing film as described in the preceding example. Transdermal nitroglycerin delivery was then determined as a function of enhancer loading as described in the Example 8 using the Minitran Patch (Riker Labs) as a control. Results are presented in Table III.

**Table III**

| Nitroglycerin Skin Flux (µg/cm²/hr)^{a} | | |
|---|---|---|
| Formulation | Oleyl Alcohol | Arlacel 80 |
| 60.0/37.5/2.5^{b} | 39.61 ± 1.94 | 39.18 ± 4.28 |
| 58.75/36.25/5.0^{b} | 41.27 ± 4.65 | 42.63 ± 7.55 |
| 57.25/35.25/7.5^{b} | 47.02 ± 2.63 | 36.04 ± 8.13 |
| Minitran Control | 31.52 ± 1.79 | 31.52 ± 1.79 |

| | | |
|---|---|---|
| ^{a}N=4 | | |
| ^{b}weight percent ratios of adhesive/nitroglycerin/enhancer | | |

In general, nitroglycerin flux was found to increase with increasing enhancer (either oleyl alcohol or Arlacel 80) content. Further, higher nitroglycerin flux values were observed with all enhancer loadings in the experimental matrix compositions than with the Minitran control.

### Example 10

The procedure of Example 9 is repeated using sorbitan monolaurate in place of sorbitan monooleate (e.g., "Arlacel® 20" from ICI Americas). Enhanced transdermal flux relative to an unenhanced nitroglycerin system is obtained.

### Example 11

The procedure of Example 9 is repeated using sorbitan monopalmitate in place of sorbitan monooleate (e.g., "Arlacel® 40" from ICI Americas). Enhanced transdermal flux relative to that obtained from with an unenhanced nitroglycerin system is obtained.

### Example 12

The procedure of Example 9 is repeated using sorbitan trioleate in place of sorbitan monooleate (e.g., "Arlacel® 85" from ICI Americas). As with the preceding examples, the transdermal flux obtained is higher than that which would be obtained for an unenhanced nitroglycerin patch.

### Example 13

The enhanced transdermal delivery of nitroglycerin from acrylic matrices containing 5 wt.% enhancer (oleyl alcohol or Arlacel® 80) was evaluated using 3 different donor skins. Enhanced nitroglycerin matrices were prepared as described in Example 9, nitroglycerin skin flux was determined as described in Example 8 using the NitroDur II and Minitran as controls. In vitro skin flux results are presented in Table X.

**Table X**

| Nitroglycerin Skin Flux (µg/cm²/hr) | | | |
|---|---|---|---|
| Formulation | Skin 1 | Skin 2 | Skin 3 |
| NitroDur II | -- | 26.06 ± 5.65 | 16.34 ± 4.64 |
| Minitran | 39.97 ± 3.69 | 44.14 ± 5.95 | 20.17 ± 1.61 |
| 5% Oleyl Alcohol^{a} | 55.99 ± 4.80 | 50.57 ± 6.53 | 30.63 ± 5.04 |
| 5% Arlacel 80^{a} | 57.45 ± 3.81 | 51.86 ± 10.72 | 26.57 ± 2.78 |

| | | | |
|---|---|---|---|
| ^{a} 58.7% wt acrylic adhesive, 36.3% wt nitroglycerin, 5.0% wt enhancer | | | |

The flux obtained from both oleyl alcohol and Arlacel 80 enhanced matrices was nearly double that obtained from the unenhanced NitroDur II control patch. Further, both enhanced matrix formulations produced consistently higher nitroglycerin skin flux than the enhanced control patch, Minitran.

### Example 14

An easy to open release tab can be prepared by overlapping two sections of the release liner by approximately 0.32 cm (1/8 inch). The adhesive/drug/enhancer solution can then be cast onto a first release liner, dried and laminated to the backing film as described in the above examples. The first release liner is then removed and the overlapping release liner is then laminated onto the adhesive surface in its place. Final transdermal patches are then die cut with the overlapping release liner section spatially within the final patch. Alternatively, the first release liner can be control depth slit following the lamination of the backing film. A section of the slit liner is then removed and a new section relaminated onto the exposed adhesive area such that the new liner section overlaps the remaining, slit prior liner which was not removed from the original laminate. Final patches are then die cut as previously described.

## Claims (Claims for the following Contracting State(s): AT, BE, CH, DE, DK, FR, GB, IT, LU, MC, NL, SE)

1. A laminated composite capable of administering a pharmacologically active agent which is a steroid, pindolol or nitroglycerin through a selected area of skin over a sustained time period, the composite comprising:
(a) a backing layer that is substantially impermeable to the pharmacologically active agent;
(b) a matrix layer having a basal surface adapted to be adhered to the skin comprising:
(i) a pressure-sensitive adhesive;
(ii) a therapeutically effective amount of the pharmacologically active agent; and
(iii) a permeation enhancer which is a sorbitan ester the structural formula: 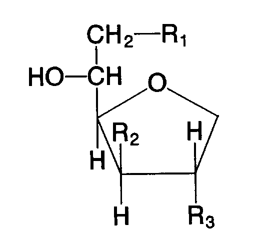 wherein R₁ has the formula -O(CO)R' where R' represents a saturated, mono-unsaturated, di-unsaturated, or tri-unsaturated aliphatic hydrocarbon group of from 7 to 21 carbon atoms optionally containing from 1 to 3 hydroxyl groups;
and both R₂ and R₃ are hydroxyl groups.

2. A composite according to claim 1 wherein the sorbitan ester is sorbitan monooleate, sorbitan monolaurate or a mixture thereof.

3. A composite according to claim 1 or 2 wherein the matrix further comprises a C₁-C₄ aliphatic alcohol.

4. A composite according to any preceding claim wherein the adhesive is a cross-linked polyacrylate polymer.

5. A composite according to any preceding claim wherein the steroid is estradiol, progesterone, norethindrone acetate or a mixture thereof.

6. A composite according to any of claims 3 to 5 wherein the C₁-C₄ aliphatic alcohol is ethanol, n-propanol, isopropanol, t-butanol or a mixture thereof.

7. A laminated composite according to any preceding claim for use in a method of treatment of the human body by therapy.

8. A process for preparing a laminated composite capable of administering a pharmacologically active agent which is a steroid, pindolol or nitroglycerin through a selected area of skin over a sustained time period, the process comprising:
(a) providing a backing layer that is substantially impermeable to the pharmacologically active agent;
(b) providing a matrix layer having a basal surface being adapted to be adhered to the skin comprising:
(i) a pressure-sensitive adhesive;
(ii) a therapeutically effective amount of the pharmacologically active agent; and
(iii) a permeation enhancer which is a sorbitan ester the structural formula: 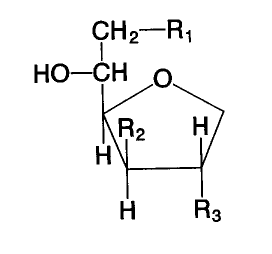 wherein R₁ has the formula -O(CO)R' where R' represents a saturated, mono-unsaturated, di-unsaturated, or tri-unsaturated aliphatic hydrocarbon group of from 7 to 21 carbon atoms optionally containing from 1 to 3 hydroxyl groups and both R₂ and R₃ are hydroxyl groups;
and bringing together the backing layer and matrix layer to form the laminated composite.

## Claims (Claims for the following Contracting State(s): ES, GR)

1. A process for preparing a laminated composite capable of administering a pharmacologically active agent which is a steroid, pindolol or nitroglycerin through a selected area of skin over a sustained time period, the process comprising:
(a) providing a backing layer that is substantially impermeable to the pharmacologically active agent;
(b) providing a matrix layer having a basal surface being adapted to be adhered to the skin comprising:
(i) a pressure-sensitive adhesive;
(ii) a therapeutically effective amount of the pharmacologically active agent; and
(iii) a permeation enhancer which is a sorbitan ester the structural formula: 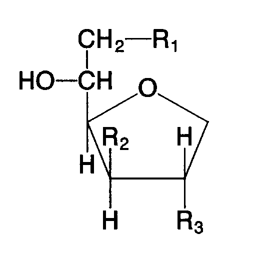 wherein R₁ has the formula -O(CO)R' where R' represents a saturated, mono-unsaturated, di-unsaturated, or tri-unsaturated aliphatic hydrocarbon group of from 7 to 21 carbon atoms optionally containing from 1 to 3 hydroxyl groups and both R₂ and R₃ are hydroxyl groups;
and bringing together the backing layer and matrix layer to form the laminated composite.

2. A process according to claim 1 wherein the sorbitan ester is sorbitan monooleate, sorbitan monolaurate or a mixture thereof.

3. A process according to claim 1 or 2 wherein the matrix further comprises a C₁-C₄ aliphatic alcohol.

4. A process according to any preceding claim wherein the adhesive is a cross-linked polyacrylate polymer.

5. A process according to any preceding claim wherein the steroid is estradiol, progesterone, norethindrone acetate or a mixture thereof.

6. A process according to any of claims 3 to 5 wherein the C₁-C₄ aliphatic alcohol is ethanol n-propanol, isopropanol, t-butanol or a mixture thereof.

7. A laminated composite preparable by a process according to any preceding claim for use in a method of treatment of the human body by therapy.

## Patentansprüche (Patentansprüche für folgende(n) Vertragsstaat(en): AT, BE, CH, DE, DK, FR, GB, IT, LU, MC, NL, SE)

1. Schichtverbundstoff, der zum Verabreichen eines pharmakologisch wirksamen Mittels, welches ein Steroid, Pindolol oder Nitroglycerin ist, durch eine ausgewählte Hautfläche über einen anhaltenden Zeitraum hinweg in der Lage ist, wobei die Zusammensetzung umfaßt:
(a) eine Rückenschicht, die im wesentlichen undurchlässig für das pharmakologisch wirksame Mittel ist;
(b) eine Matrixschicht mit einer Grundseite, die zum Haften auf der Haut vorgesehen ist, umfassend:
(i) einen druckempfindlichen Klebstoff;
(ii) eine therapeutisch wirksame Menge des pharmakologisch wirksamen Mittels; und
(iii) ein Durchlässigkeits-verbesserndes Mittel, welches ein Sorbitanester der folgenden Strukturformel ist: 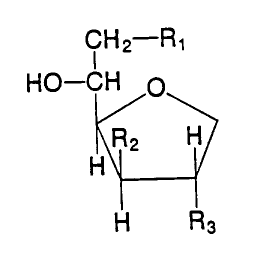 wobei R₁ die Formel -O(CO)R' aufweist, worin R' für eine gesättigte, mono-ungesättigte, di-ungesättigte oder tri-ungesättigte aliphatische Kohlenwasserstoffgruppe mit 7 bis 21 Kohlenstoffatomen steht, die wahlweise 1 bis 3 Hydroxylgruppen enthält;

2. Verbundstoff nach Anspruch 1, wobei der Sorbitanester Sorbitanmonooleat, Sorbitanmonolaurat oder ein Gemisch davon ist.

3. Verbundstoff nach Anspruch 1 oder 2, wobei die Matrix außerdem einen C₁-C₄-aliphatischen Alkohol umfaßt.

4. Verbundstoff nach einem der vorangehenden Ansprüche, wobei der Klebstoff ein vernetztes Polyacrylat-Polymer ist.

5. Verbundstoff nach einem der vorangehenden Ansprüche, wobei das Steroid Estradiol, Progesteron, Norethindronacetat oder ein Gemisch davon ist.

6. Verbundstoff nach einem der Ansprüche 3 bis 5, wobei der C₁-C₄-aliphatische Alkohol Ethanol, n-Propanol, Isopropanol, t-Butanol oder ein Gemisch davon ist.

7. Schichtverbundstoff nach einem der vorangehenden Ansprüche zur Verwendung bei einer Methode zur Behandlung des menschlichen Körpers durch Therapie.

8. Verfahren zur Herstellung eines Schichtverbundstoffs, der zum Verabreichen eines pharmakologisch wirksamen Mittels, welches ein Steroid, Pindolol oder Nitroglycerin ist, durch eine ausgewählte Hautfläche über einen anhaltenden Zeitraum hinweg in der Lage ist, wobei das Verfahren umfaßt:
(a) Bereitstellen einer Rückenschicht, die im wesentlichen undurchlässig für das pharmakologisch wirksame Mittel ist;
(b) Bereitstellen einer Matrixschicht mit einer Grundseite, die zum Haften auf der Haut vorgesehen ist, umfassend:
(i) einen druckempfindlichen Klebstoff;
(ii) eine therapeutisch wirksame Menge des pharmakologisch wirksamen Mittels; und
(iii) ein Durchlässigkeits-verbesserndes Mittel, welches ein Sorbitanester der folgenden Strukturformel ist: 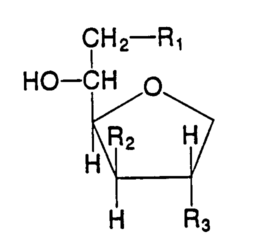 wobei R₁ die Formel -O(CO)R' aufweist, worin R' für eine gesättigte, mono-ungesättigte, di-ungesättigte oder tri-ungesättigte aliphatische Kohlenwasserstoffgruppe mit 7 bis 21 Kohlenstoffatomen steht, die wahlweise 1 bis 3 Hydroxylgruppen enthält, wobei R₂ und R₃ Hydroxylgruppen sind;
und Zusammenbringen der Rückenschicht und der Matrixschicht zur Erzeugung des Schichtverbundstoffs.

## Patentansprüche (Patentansprüche für folgende(n) Vertragsstaat(en): ES, GR)

1. Verfahren zur Herstellung eines Schichtverbundstoffs, der zum Verabreichen eines pharmakologisch wirksamen Mittels, welches ein Steroid, Pindolol oder Nitroglycerin ist, durch eine ausgewählte Hautfläche über einen anhaltenden Zeitraum hinweg in der Lage ist, wobei das Verfahren umfaßt:
(a) Bereitstellen einer Rückenschicht, die im wesentlichen undurchlässig für das pharmakologisch wirksame Mittel ist;
(b) Bereitstellen einer Matrixschicht mit einer Grundseite, die zum Haften auf der Haut vorgesehen ist, umfassend:
(i) einen druckempfindlichen Klebstoff;
(ii) eine therapeutisch wirksame Menge des pharmakologisch wirksamen Mittels; und
(iii) ein Durchlässigkeits-verbesserndes Mittel, welches ein Sorbitanester der folgenden Strukturformel ist: 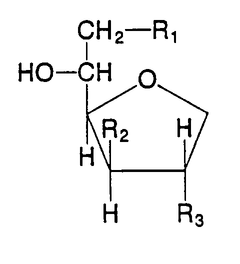 wobei R₁ die Formel -O(CO)R' aufweist, worin R' für eine gesättigte, mono-ungesättigte, di-ungesättigte oder tri-ungesättigte aliphatische Kohlenwasserstoffgruppe mit 7 bis 21 Kohlenstoffatomen steht, die wahlweise 1 bis 3 Hydroxylgruppen enthält, wobei R₂ und R₃ Hydroxylgruppen sind;
und Zusammenbringen der Rückenschicht und der Matrixschicht zur Erzeugung des Schichtverbundstoffs.

2. Verfahren nach Anspruch 1, wobei der Sorbitanester Sorbitanmonooleat, Sorbitanmonolaurat oder ein Gemisch davon ist.

3. Verfahren nach Anspruch 1 oder 2, wobei die Matrix außerdem einen C₁-C₄-aliphatischen Alkohol umfaßt.

4. Verfahren nach einem der vorangehenden Ansprüche, wobei der Klebstoff ein vernetztes Polyacrylat-Polymer ist.

5. Verfahren nach einem der vorangehenden Ansprüche, wobei das Steroid Estradiol, Progesteron, Norethindronacetat oder ein Gemisch davon ist.

6. Verfahren nach einem der Ansprüche 3 bis 5, wobei der C₁-C₄-aliphatische Alkohol Ethanol, n-Propanol, Isopropanol, t-Butanol oder ein Gemisch davon ist.

7. Schichtverbundstoff, herstellbar mittels eines Verfahrens nach einem der vorangehenden Ansprüche, zur Verwendung bei einer Methode zur Behandlung des menschlichen Körpers durch Therapie.

## Revendications (Revendications pour l'(les) Etat(s) contractant(s) suivant(s): AT, BE, CH, DE, DK, FR, GB, IT, LU, MC, NL, SE)

1. Stratifié permettant d'administrer un agent pharmacologiquement actif qui est un stéroïde, du pindolol ou de la nitroglycérine à travers une zone de peau déterminée pendant une période de temps continu, le stratifié comprenant :
(a) une couche support qui est essentiellement imperméable à l'agent pharmacologiquement actif,
(b) une couche de matrice ayant une surface de base apte à adhérer à la peau comprenant :
(i) un adhésif sensible à la pression,
(ii) une quantité thérapeutiquement efficace d'un agent pharmacologiquement actif et
(iii) un renforçateur de pénétration qui est un ester de sorbitan ayant la formule : 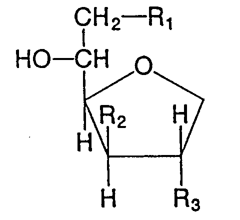 dans laquelle R₁ a la formule -O(CO)R', R' représentant un groupe hydrocarboné aliphatique saturé, mono-insaturé, di-insaturé ou tri-insaturé ayant de 7 à 21 atomes de carbone contenant éventuellement de 1 à 3 groupes hydroxyles ;
et R₂ et R₃ représentent tous deux des groupes hydroxyles.

2. Stratifié selon la revendication 1 dans lequel l'ester de sorbitan est le monooléate de sorbitan, le monolaurate de sorbitan ou un mélange de ceux-ci.

3. Stratifié selon la revendication 1 ou 2 dans lequel la matrice contient en outre un alcool aliphatique en C₁-C₄.

4. Stratifié selon l'une quelconque des revendications précédentes dans lequel l'adhésif est un polymère polyacrylate réticulé.

5. Stratifié selon l'une quelconque des revendications précédentes dans lequel le stéroïde est l'oestradiol, la progestérone, l'acétate de norethindrone ou un mélange de ceux-ci.

6. Stratifié selon l'une des revendications 3 à 5 dans lequel l'alcool aliphatique C₁-C₄ est l'éthanol, le n-propanol, l'isopropanol, le t-butanol ou un mélange de ceux-ci.

7. Stratifié selon l'une quelconque des revendications précédentes pour son utilisation dans un procédé de traitement thérapeutique du corps humain.

8. Procédé pour la préparation d'un stratifié permettant d'administrer un agent pharmacologiquement actif qui est un stéroïde, du pindolol ou de la nitroglycérine sur une zone de peau déterminée pendant une période de temps continu, le procédé consistant a :
(a) fournir une feuille support qui est essentiellement imperméable à l'agent pharmacologiquement actif,
(b) fournir une couche de matrice ayant une surface de base apte à adhérer à la peau comprenant :
(i) un adhésif sensible à la pression ;
(ii) une quantité thérapeutiquement active de l'agent pharmacologiquement actif et
(iii) un renforçateur de pénétration qui est un ester de sorbitan de formule : 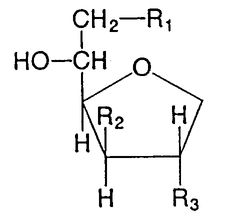 dans laquelle R₁ a la formule -O(CO)R', R' représente un groupe hydrocarboné aliphatique saturé, mono-insaturé, di-insaturé ou tri-insaturé ayant de 7 à 21 atomes de carbone contenant éventuellement de 1 à 3 groupes hydroxyles et R₂ et R₃ représentent tous deux des groupes hydroxyles,
et à assembler la couche support et la couche de matrice pour former le stratifié.

## Revendications (Revendications pour l'(les) Etat(s) contractant(s) suivant(s): ES, GR)

1. Procédé pour la préparation d'un stratifié apte à administrer un agent pharmacologiquement actif qui est un stéroïde, du pindolol ou de la nitroglycérine sur une zone de peau déterminée pendant une période de temps continu, le procédé consistant à :
(a) fournir une couche support qui est essentiellement imperméable à l'agent pharmacologiquement actif,
(b) fournir une couche de matrice ayant une surface de base apte à adhérer à la peau comprenant :
(i) un adhésif sensible à la pression ;
(ii) une quantité thérapeutiquement efficace d'un agent pharmacologiquement actif ; et
(iii) un renforçateur de pénétration qui est un ester de sorbitan de formule : 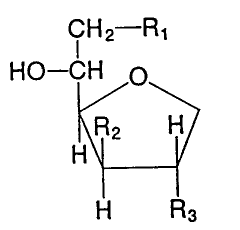 dans laquelle R₁ a la formule -O(CO)R', R' représentant un groupe aliphatique hydrocarboné saturé, mono-insaturé, di-insaturé ou tri-insaturé ayant de 7 à 21 atomes de carbone contenant éventuellement de 1 à 3 groupes hydroxyles et R₂ et R₃ représentent tous deux des groupes hydroxyles,
et à assembler la couche support et la couche de matrice pour former le stratifié.

2. Procédé selon la revendication 1 dans lequel l'ester de sorbitan et le monooléate de sorbitan, le monolaurate de sorbitan ou un mélange de ceux-ci.

3. Procédé selon la revendication 1 ou 2 dans lequel la matrice comprend en outre un alcool aliphatique en C₁-C₄.

4. Procédé selon l'une quelconque des revendications précédentes dans lequel l'adhésif est un polymère polyacrylate réticulé.

5. Procédé selon l'une quelconque des revendications précédentes dans lequel le stéroïde est l'oestradiol, la progestérone, l'acétate de norethindrone ou un mélange de ceux-ci.

6. Procédé selon l'une quelconque des revendications 3 à 5 dans lequel l'alcool aliphatique en C₁-C₄ est l'éthanol, le n-propanol, l'isopropanol, le t-butanol ou un mélange de ceux-ci.

7. Stratifié susceptible d'être obtenu par un procédé selon l'une quelconque des revendications précédentes pour son utilisation dans une méthode de traitement thérapeutique du corps humain.
